# EUROPÄISCHE PATENTANMELDUNG

(11) **EP 2 669 559 A2**
(43) Veröffentlichungstag der Anmeldung: **04.12.2013**
(21) Anmeldenummer: 13000482.3
(22) Anmeldetag: 31.01.2013
(51) Int. Cl.: F16K 31/08

(54) **Ventil sowie dessen Verwendung**

(30) Priorität: 30.05.2012 DE 102012010615
(71) Anmelder: Infors AG, 4103 Bottmingen (CH)
(72) Erfinder: Hawrylenko, Alexander, CH-4103 Bottmingen (CH)
(74) Vertreter: Säger, Manfred

(57) **Zusammenfassung**

Ein zwei Schaltstellungen aufweisendes Ventil (5) mit einem Gehäuse (6), mit einem Eingang (7) sowie einem Ausgang (8) für ein Fluid und mit einem zwischen dem Eingang (7) und dem Ausgang (8) in dem Gehäuse (6) angeordneten Kolben (9), der in seiner der einen Schaltstellung des Ventils (5)entsprechenden Startlage den Durchgang des Fluids zwischen dem Eingang (7) und dem Ausgang (8) sperrt und dieses in seiner der anderen Schaltstellung des Ventils entsprechenden Schaltlage durchlässt, kennzeichnet sich dadurch aus, dass der Kolben (9) von der einen Kraft eines Permanent-Magneten (13) in seiner Startlage entgegen der demgegenüber um ein ΔF geringeren anderen Kraft einer Feder (14) gehalten ist und dass der Kolben (9) bei Einwirkung einer in Richtung der anderen Kraft wirkenden, grösser als ΔF bemessenen weiteren Kraft von seiner Startlage in die Schaltlage bewegt wird und in dieser dort aufgrund der anderen Kraft verbleibt.

## Beschreibung

Die Erfindung betrifft ein gattungsgemässes Ventil nach dem Oberbegriff des des Hauptanspruchs, nämlich ein solches, zwei Schaltstellungen aufweisendes Ventil mit einem Gehäuse, mit einem Eingang sowie einem Ausgang für ein Fluid und mit einem zwischen dem Eingang und dem Ausgang in dem Gehäuse angeordneten Kolben, der in seiner der einen Schaltstellung des Ventils entsprechenden Startlage den Durchgang des Fluids zwischen dem Eingang und dem Ausgang sperrt und dieses in seiner der anderen Schaltstellung des Ventils entsprechenden Schaltlage durchlässt. Auuserdem betrifft die Erfindung die Verwendung des Ventils.

Ein solches gattungsgemässes Ventil ist in einer Vielzahl von Ausführungsformen bekannt. Allen ist gemeinsam, dass sie nur aufwendig in die eine Schaltstellung gebracht werden kann.

Der Erfindung liegt daher die Aufgabe zugrunde, ein weniger aufwendig arbeitendes Ventil bereitzustellen. Diese Aufgabe wird bei einem gattungsgemässen Ventil nach dem Oberbegriff des Hauptanspruchs erfindungsgemäss durch dessen kennzeichnende Merkmale, also dadurch gelöst, dass der Kolben von der einen Kraft eines Permanent-Magneten in seiner Startlage entgegen der demgegenüber um ein ΔF geringeren anderen Kraft einer Feder gehalten ist und dass der Kolben bei Einwirkung einer in Richtung der anderen Kraft wirkenden, grösser als ΔF bemessenen weiteren Kraft von seiner Startlage in die Schaltlage bewegt wird und in dieser dort aufgrund der anderen Kraft verbleibt.

So wird der Kolben des Ventils erfindungsgemäss von seiner magnetischen einen Kraft in seiner Startlage gehalten, in welcher Schaltstellung das Ventil gesperrt ist, so dass sein Aus- von seinem Eingang getrennt ist. Diese eine Kraft ist nur um ΔF grösser als die von der Feder bewirkte andere Kraft. Wird nun auf den Kolben eine ΔF übersteigende weitere Kraft bewirkt, so wird die magnetische eine Kraft überwunden und der Kolben bewegt sich etwas aus seiner Startlage. Dabei nimmt die magnetische eine Kraft, bezogen auf die lineare andere Kraft der Feder überproportional ab, verringert sich also stark und sobald sie sich um ΔF verkleinert hat, drückt die Feder den Kolben in Richtung seiner Schalt- und Endlage, wo er verbleibt und in der das Ventil in seiner anderen Schaltstellung den Eingang an dem Kolben vorbei mit dem Ausgang verbindet. Die weitere Kraft kann auf eine Vielzahl von Arten erzeugt werden. So kann z.B. ein das Ventil von aussen umgebender magnetischer Ring betätigt werden, um die weitere Kraft zu erzeugen. Auch kann der Kolben so ausgestaltet und bemessen sein, dass lageabhängig die magnetische eine Kraft grösser als die andere Kraft der Feder, weil diese um das Gewicht des Kolbens verkleinert wird, z.B. wenn das Ventil auf den Kopf gestellt wird. Dreht man es wieder zurück, so addiert sich das Gewicht des Kolbens (grösser als ΔF) als weitere Kraft zu der anderen Kraft der Feder hinzu, wodurch der Kolben in seine Schaltlage bewegt wird.

Mit besonderem Vorteil wird aber erfindungsgemäss die weitere Kraft bei Beaufschlagung des Eingangs mit dem unter einem Überdruck stehenden Fluid unter Bewegung des Kolbens von seiner Startlage in die Schaltlage erzeugt.

Das Ventil der Erfindung lässt sich auch mit ganz besonderem Vorteil an (je)dem Gasanschluss eines Fermenters zum Züchten von Mikroorganismen wie Bakterien- oder Pilzkulturen oder Zellkulturen verwenden, insbesondere wenn das Ventil mit seinem Ausgang an den Gasanschluss des Fermenters angeschlossen ist und dann der Eingang des Ventils als eigentlicher Gasanschluss des Fermenters dient, an den auch ein Sterilfilter anschliessbar ist.

Wenn das durch Autoklavierung sterile Ventil mit seinem Kolben in der Startlage also an den Fermenter ansgeschlossen ist, kann dieser durch Zugabe von Lauge, z.B. Natronlauge und/oder Wasser gereinigt werden, so kann bei Vorsehen eines Reinigungsanschlusses zwischen dem Kolben und dem Ausgang auch dieser Teil des Ventils gereinigt werden. Beim Reinigung wird bei dem Reinigungsvorgang das Gemisch ggf. unter Umkehr seiner Fliessrichtung und ggf. Zugabe von Wasser von dem Wasseranschluss ausreichend lange zirkuliert, danach durch Zugabe von Säure, z.B. Phosphorsäure neutralsiert und schliesslich durch Zugabe von Luft in den Gasraum über den Entnahmeanschluss des Laborfermenters zu dem Tauchrohranschluss und letztlich zu dem Abwasseranschluss transportiert, der an einen sogenannten Killtank angeschlossen werden kann. Nach dem Ende des Reinigungsvorganges können alle Schlauchanschlüsse zu dem Laborfermenter an den Reinigungsanschlüssen unter Belassung der Ventile noch in deren Reinigungsstartstellung an dem Laborfermenter getrennt und ggf. verschlossen werden, der dann für ein neuen Fementierzyklus vollständig gereinigt bereit steht. Wird der Fermenter dann zu dem neuen Fementierzyklus über die Ventile begast, so werden deren Kolben alleine dadurch, dass das unter Überdruck stehende Fluid an dem Eingang ansteht, von der Start- in die Schaltlage bewegt, ohne dass die Reinigung seine Sterilität beeinträchtigt hat.

Weitere zweckmässige Ausgestaltungen und Weiterbildungen der Erfindung sind in den Unteransprüchen gekennzeichnet.

Ein Ausführungsbeispiel der Erfindung wird nachfolgend unter Bezugnahme auf die Zeichnung erläutert. In dieser zeigt:
- Figur 1: das erfindungsgemässe Ventil in der einen Schaltstellung, im Querschnitt und
- Figur 2: das Ventil nch Figur 1 in der anderen Schaltstellung, im Querschnitt.

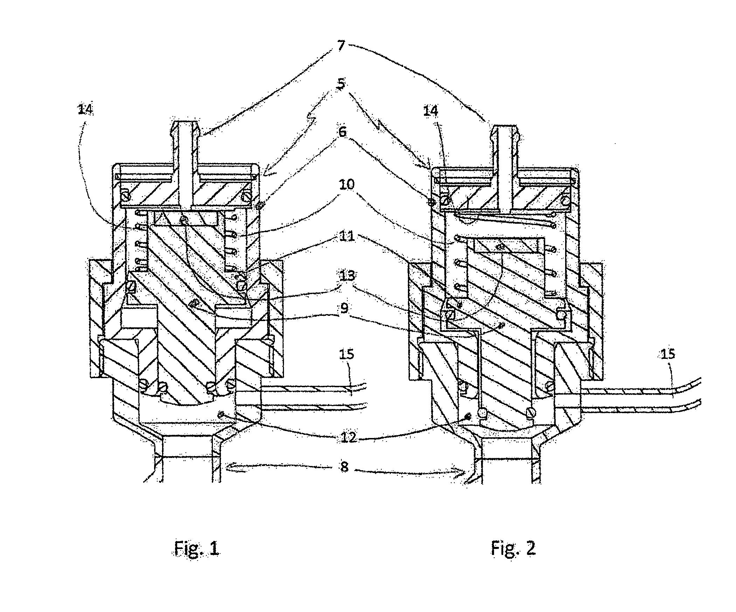

Das erfindungsgemässe Ventil 5 mit seinen zwei Schaltstellungen weist ein Gehäuse 6 mit einem jeweils als Nippel ausgebildeten Eingang 7 sowie einem Ausgang 8 für ein Fluid und mit einem zwischen dem Eingang 7 und dem Ausgang 8 in dem Gehäuse 6 angeordneten Kolben 9 auf. Dieser sperrt in seiner der einen Schaltstellung des Ventils entsprechenden Startlage den Durchgang des Fluids zwischen dem Eingang 7 und dem Ausgang 8, wie in Figur 1 dargestellt. Wie Figur 2 zeigt, wird in seiner der anderen Schaltstellung des Ventils entsprechenden Schaltlage das Fluid durchgelassen.

Den Kolben 9 umgibt eine Feder 10, die sich einerseits an einem Absatz 11 des Kolbens, andererseits an der Innenseite des den Eingang 7 aufweisenden Gehäuseoberteils, die dem Kolben in seiner Startlage zugewandt ist, abstützt und -unter Bewirkung einer anderen Kraft der Feder auf den Kolben- vorgespannt ist. Das dem Gehäuseoberteil abgewandte Gehäuseunterteil weist den Ausgang 8 auf. Der Raum 12 zwischen dem Ausgang 8 und dem Kolben 9 kann an eine Reinigungsleitung 13 angeschlossen sein, die aber auch entfallen kann. Der Kolben 9, das Gehäuseober- und -unterteil weisen ferner an sich bekannte und daher hier nicht näher beschriebene Dichtungen, z.B. in Form von O-Ringen auf.

Der Kolben 9 ist beim wiedergegebenen Ausführungsbeispiel auf seiner in der Startlage dem -den Eingang aufweisenden- Gehäuseoberteil zugewandten Seite mit einem in eine Ausnehmung eingesetzten zylinderförmigen Permanent-Magnet 13 versehen. Das Gehäuse 6 ist auf der in der Startlage (Figur 1) dem Kolben 9 zugewandten Seite mit einer Scheibe 14 aus einem magnetisch leitenden Werkstoff versehen; das Gehäuse 6 und der Kolben 9 sind aus einem magnetisch nicht leitenden Werkstoff. Zwischen der Scheibe 14 und dem Permanent-Magnet 13 wirkt dessen magnetische Kraft. Unter der Scheibe 14 ist ein bis zu dem Eingang 7 reichender Kanal zum Durchlass des Fluids vorgesehen.

Der Kolben 9 ist dabei von der einen Kraft des Permanent-Magneten 14 in seiner Startlage (Figur 1) entgegen der demgegenüber um ein ΔF geringeren anderen Kraft der Feder 10 gehalten. Erst wenn auf den Kolben 9 eine weitere Kraft einwirkt, die grösser als ΔF bemessenen ist, wird er (9) von seiner Startlage (Figur 1) in die Schaltlage (Figur 2) bewegt und in dieser dort aufgrund der anderen Kraft gehalten. Diese ΔF übersteigende weitere Kraft bewirkt, dass die magnetische -eine- Kraft überwunden und der Kolben 9 etwas aus seiner Startlage bewegt wird. Dabei nimmt die magnetische eine Kraft, bezogen auf die lineare andere Kraft der Feder 10 überproportional ab, verringert sich also stark und sobald sie sich um ΔF verkleinert hat, drückt die Feder 10 den Kolben 9 in Richtung seiner Schalt- und Endlage, wo er verbleibt und in der das Ventil in seiner anderen Schaltstellung den Eingang 7 an dem Kolben 9 vorbei über den Raum 12 mit dem Ausgang 8 verbindet. Dies geschieht vom Eingang 7 über den unter der Scheibe 14 verlaufenden Kanal, dann über eine Verbreiterung des Kolbenraums unterhalb des Absatzes 11 des Kolbens 9, von der der Absatz bei der Bewegung in die in Figur 2 gezeigte Schaltlage freikommt und schliesslich durch das Spiel um den unteren Teil des Kolbens 9, wenn dessen dort angebrachte Dichtung in Form des O-Rings in den Raum 12 zwischen dem Ausgang 8 und dem Kolben 9 gelangt ist.

Die weitere Kraft kann, wie schon dargelegt, auf eine Vielzahl von Arten erzeugt werden. Beim wiedergegebenen Ausführungsbeispiel wird die weitere Kraft bei Beaufschlagung des Eingangs 7 mit dem unter einem Überdruck stehenden Fluid in Form vorzugsweise steriler Luft unter Bewegung des Kolbens 9 von seiner Startlage in die Schaltlage erzeugt.

Vor seinem Einbau bzw. Einsatz sollte das Ventil 5 autoklaviert und damit sterilisiert worden sein.

## Patentansprüche

1. Zwei Schaltstellungen aufweisendes Ventil (5) mit einem Gehäuse (6), mit einem Eingang (7) sowie einem Ausgang (8) für ein Fluid und mit einem zwischen dem Eingang und dem Ausgang in dem Gehäuse angeordneten Kolben (9), der in seiner der einen Schaltstellung des Ventils entsprechenden Startlage den Durchgang des Fluids zwischen dem Eingang und dem Ausgang sperrt und dieses in seiner der anderen Schaltstellung des Ventils entsprechenden Schaltlage durchlässt,
**dadurch gekennzeichnet, dass** der Kolben (9) von der einen Kraft eines Permanent-Magneten (13) in seiner Startlage (Figur 1) entgegen der demgegenüber um ein ΔF geringeren anderen Kraft einer Feder (10) gehalten ist und dass der Kolben bei Einwirkung einer in Richtung der anderen Kraft wirkenden, grösser als ΔF bemessenen weiteren Kraft von seiner Startlage in die Schaltlage (Figur 2) bewegt wird und in dieser dort aufgrund der anderen Kraft verbleibt.

2. Ventil nach Anspruch 1, **dadurch gekennzeichnet, dass** der Kolben (9) auf der in seiner Startlage dem den Eingang aufweisenden Gehäuseoberteils zugewandten Seite mit dem Permanent-Magnet (13) oder umgekehrt versehen ist.

3. Ventil nach Anspruch 2, **dadurch gekennzeichnet, dass** sowohl der Kolben (9) auf der in seiner Startlage dem Gehäuseoberteil zugewandten Seite als auch dieses mit einem entsprechend anziehend gepolten Permanent-Magnet versehen ist.

4. Ventil nach Anspruch 2, **dadurch gekennzeichnet, dass** der Kolben (9) auf der in seiner Startlage dem Gehäuseoberteil zugewandten Seite mit einer Scheibe (14) aus einem magnetisch leitenden Werkstoff oder umgekehrt versehen ist.

5. Ventil nach einem der vorhergehenden Ansprüche, **dadurch gekennzeichnet, dass** das Gehäuse (6) und/oder der Kolben (9) aus einem magnetisch nicht leitenden Werkstoff ausgebildet ist.

6. Ventil nach einem der vorhergehenden Ansprüche, **dadurch gekennzeichnet, dass** die weitere Kraft bei Beaufschlagung des Eingangs mit dem unter einem Überdruck stehenden Fluid unter Bewegung des Kolbens (9) von seiner Startlage in die Schaltlage erzeugt wird.

7. Ventil nach einem der vorhergehenden Ansprüche, **dadurch gekennzeichnet, dass** es autoklavierbar ausgebildet ist.

8. Verwendung eines Ventils (5) nach einem der Ansprüche 1 bis 7 an dem Gasanschluss eines Fermenters zum Züchten von Mikroorganismen wie Bakterien- oder Pilzkulturen oder Zellkulturen.

9. Verwendung nach Anspruch 8, **dadurch gekennzeichnet, dass** das Ventil (5) mit seinem Ausgang (8) an den Gasanschluss des Fermenters angeschlossen ist.

10. Verwendung nach Anspruch 9, **dadurch gekennzeichnet, dass** der Eingang (7) des Ventils (5) als Gasanschluss des Fermenters dient.
